# EUROPEAN PATENT APPLICATION

(11) **EP 1 544 655 A2**
(43) Date of publication of application: **22.06.2005**
(21) Application number: 04029348.2
(22) Date of filing: 10.12.2004
(51) Int. Cl.: G02B 21/34, B01L 3/00

(54) **Patch for microarray reaction chamber having adhesive means support and two or more adhesive materials**

(30) Priority: 17.12.2003 KR 2003092568
(71) Applicant: Samsung Electronics Co., Ltd., Suwon-si, Gyeonggi-do (KR)
(72) Inventor: Lee, Jeong-gun, Gunpo-si, Gyeonggi-do (KR); Lee, Hun-joo, Jongno-gu, Seoul (KR)
(74) Representative: Grünecker, Kinkeldey, Stockmair & Schwanhäusser Anwaltssozietät

(57) **Abstract**

Provided is a patch for a reaction chamber of a microarray, which includes a support means provided with a sample inlet and an adhesive means adhering to the support means and providing an adhesive strength capable of being attached to a microarray substrate, wherein the adhesive means has stacked adhesive means supports having both surfaces to which an adhesive material is deposited and a first adhesive material located between the adhesive means support and the adhesive means support or between the adhesive means support and the support means has 33 oz/in greater adhesive strength (according to ASTM D3330 Method), based on an adhesive strength to steel, than a second adhesive material located between the patch and the microarray substrate.

## Description

### BACKGROUND OF THE INVENTION

### 1. Field of the Invention

The present invention relates to a patch for a reaction chamber of a microarray and a method of using the same.

### 2. Description of the Related Art

Generally, a "microarray" indicates that a group of polymers, such as polynucleotides or proteins, is tightly immobilized on a solid substrate and the group of polymers is immobilized at a specific region. Such microarray is well known in the art. Regarding the microarray, see, for example, U.S. Patent Nos. 5,445,934 and 5,744,305. Examples of the microarray include a protein array and a polynucleotide array. "A reaction chamber of a microarray" is a space that is provided with constant conditions for performing a reaction on the microarray. The reaction chamber of a conventional microarray is not ready made but is formed by binding a microarray substrate and a coverglass or a patch just before performing a reaction.

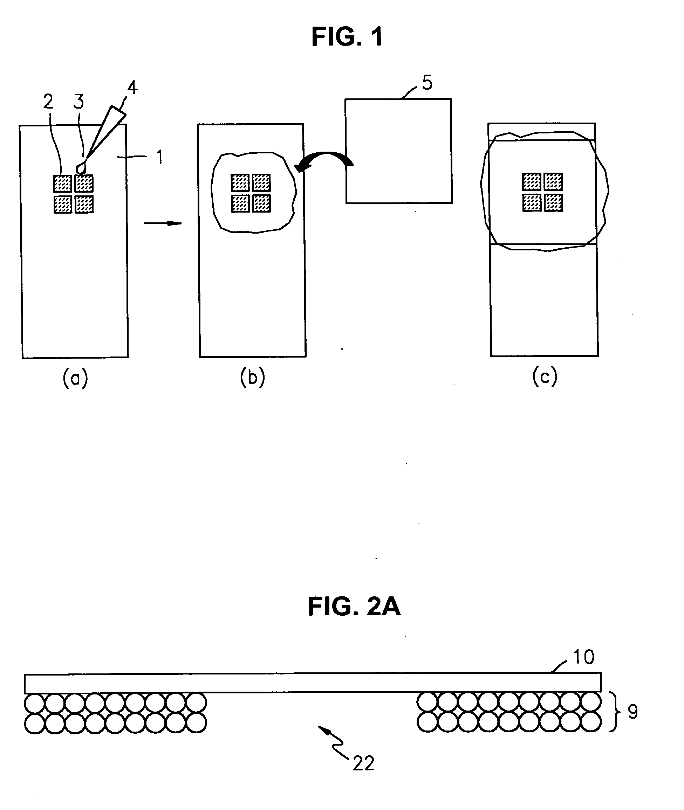

An example of a process of forming a reaction chamber using a coverglass is described with reference to FIG. 1. An appropriate amount of a sample 3 containing a target polynucleotide is dropped using a micropipet 4 onto a polynucleotide array 1 on which a probe polynucleotide 2 is immobilized. A coverglass 5 is placed on the sample droplet 3. By this measure, a space between the coverglass 5 and a microarray substrate functions as a reaction chamber. This reaction chamber can be a place performing hybridization under hybridization conditions.

Since the method uses the coverglass 5, the required cost is low. However, it is not easy to control the amount of a sample to be added, and evaporation of the sample may occur at high temperature and unsaturated relative humidity. Signals including an optical signal obtained as a reaction result are affected by the evaporation of the sample, so it is difficult to obtain reproducible results.

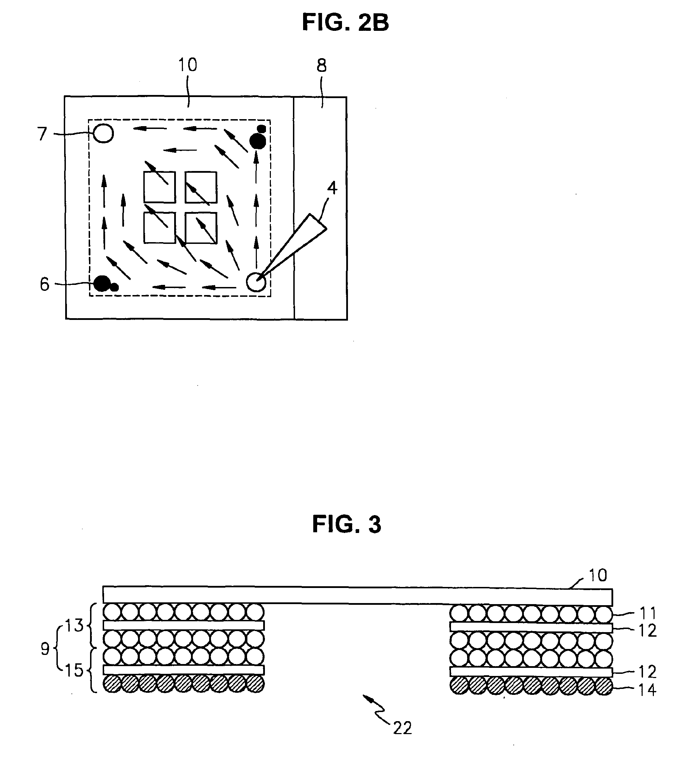

A method of forming a reaction chamber using a conventional patch is also known in the art. The patch consists of an adhesive means adhering to a substrate and a support means to which the adhesive means attached. Adherence of the patch to the substrate provides a chamber-forming portion, which is a space for forming the reaction chamber. A patch for a reaction chamber of a microarray and a method of forming the same are described with reference to FIG. 2. FIG. 2A is a side cross-sectional view of a patch, which is composed of a support means 10 provided with a sample inlet and an adhesive means 9 attached thereto and has a reaction chamber-forming portion 22. FIG. 2B is a plan perspective view of a reaction chamber when adding a reaction mixture to the reaction chamber formed by attaching the patch of FIG. 2A to a substrate. The reaction chamber is formed by attaching the patch to the substrate using a handle 8. In order to perform a reaction in the formed reaction chamber, a sample is injected into a sample inlet 7 using a micropipet 4. At this time, a conventional reaction chamber has a drawback in that the shape of the chamber-forming portion is not optimized and so bubbles 6 are produced. Since the patch uses only one type of adhesive material, a leakage can be prevented by using a material that strongly adheres to the substrate. However, it is difficult to detach the patch from the substrate after completing the reaction, and the adhesive material can remain even though the patch has been detached. Since the adhesive means 9 weakly adheres to the support means 10, the adhesive means 9 may be separated from the support means 10. Although the capacity of the chamber can be controlled by increasing the thickness of the adhesive means, it is difficult to obtain a uniform thickness when increasing the thickness since only one type of adhesive material is used.

The inventors of the present invention have conducted an intensive study of a patch for a microarray reaction chamber which can be easily detached from a microarray substrate, prevent a support means and an adhesive means from being separated, and stably control the thickness of the adhesive means, based on conventional technologies as described above, and found that the above effects can be obtained by introducing an adhesive means support into the adhesive means and using two or more adhesive materials, thereby completing the present invention.

### SUMMARY OF THE INVENTION

The present invention provides a patch for a microarray reaction chamber which can be easily detached from a microarry substrate, prevent a support means and an adhesive means from being separated, and stably control the thickness of the adhesive means.

The present invention also provides a method of using the patch for a microarray reaction chamber.

According to an aspect of the present invention, there is provided a patch for a reaction chamber of a microarray, which includes a support means provided with a sample inlet and an adhesive means adhering to the support means and providing an adhesive strength capable of being attached to a microarray substrate and has a chamber-forming portion defined by the support means and the adhesive means,
wherein the adhesive means has stacked adhesive means supports having both surfaces to which an adhesive material is deposited and a first adhesive material located between the adhesive means supports and the adhesive means supports or between the adhesive means support and the support means has 33 oz/in greater adhesive strength (according to ASTM D3330 Method), based on an adhesive strength to steel, than a second adhesive material located between the patch and the microarray substrate.

According to another aspect of the present invention, there is provided a method of using the patch of the present invention, the method comprising:
attaching the patch of any one of claims 1 through 8 on a microarray substrate having an immobilized biomolecule, so as to form a reaction chamber; and
injecting a reaction mixture into the reaction chamber so as to perform a reaction.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates an example of a process of forming a chamber using a conventional coverglass;
FIG. 2A is a side cross-sectional view of a conventional patch for forming a chamber;
FIG. 2B illustrates an example of a process of forming a chamber using a conventional patch;
FIG. 3 is a side cross-sectional view of a patch for a microarray reaction chamber of the present invention;
FIG. 4 illustrates a process of forming an adhesive means of the patch of the present invention;
FIG. 5 illustrates a process of forming a reaction chamber-forming portion from an adhesive means of the present invention;
FIGS. 6 and 7 illustrate examples of a method of using the patch of present invention;
FIG. 8 illustrates an adhesive means of a patch used in Example 2 of the present invention; and
FIG. 9 illustrates a process of forming a chamber-forming portion from an adhesive means of Example 2 of the present invention.

### DETAILED DESCRIPTION OF THE INVENTION

The present invention provides a patch for a reaction chamber of a microarray, which includes a support means provided with a sample inlet and an adhesive means adhering to the support means and providing an adhesive strength allowing the patch to attach to a microarray substrate and has a chamber-forming portions defined by the support means and the adhesive means, wherein the adhesive means has stacked adhesive means supports having both surfaces to which an adhesive material is deposited and a first adhesive material located between the adhesive means support and the adhesive means support or between the adhesive means support and the support means has 33 oz/in greater adhesive strength (according to ASTM D3330 Method), based on an adhesive strength to steel, than a second adhesive material located between the patch and the microarray substrate.

The support means may be composed of a hydrophobic material having a contact angle of 80° or greater. The hydrophobic material includes hydrophobic plastic or hydrophobically-modified silicone. Examples of the hydrophobic material include, but are not limited to, polyethylene, polypropylene, polycarbonate, polystyrene, polymethylmethacrylate (PMMA), polydimethylsiloxane (PDMS), polychlorinated biphenyl (PCB), hydrophobically-modified silicon wafer, hydrophobically-modified_ITO (indium tin oxide) glass, and a combination thereof.

Examples of the adhesive means support include, but are not limited to, polyethyleneterephthalate (PET), polyethylene, and polyester. One or more adhesive means supports may be used according to the purpose of using the patch, and materials thereof may be identical or different.

The first adhesive material attaches the adhesive means supports or attaches the adhesive means support and the support means. The first adhesive material is any material having 33 oz/in greater adhesive strength than the second adhesive material. The first adhesive material may have an adhesive strength of 45 oz/in or greater.

The second adhesive material attaches the patch and the microarray substrate. The second adhesive material has 33 oz/in less adhesive strength than the first adhesive material. The second adhesive material has repositionability and has an adhesive strength of 12-25 oz/in.

The present invention also provides a method of using the patch according to the present invention, the method including: attaching the patch according to the present invention on a microarray substrate having an immobilized biomolecule so as to form a reaction chamber; and injecting a reaction mixture into the reaction chamber so as to perform a reaction.

"The biomolecule microarray" herein indicates that a group of biomolecules, such as polynucleotides or proteins, is tightly immobilized at a specific region on a substrate. Such microarray is well known in the art. Regarding the microarray, see, for example, U.S. Patent Nos. 5,445,934 and 5, 744,305. Examples of the microarray include a protein array and a polynucleotide array.

When the microarray is a polynucleotide array, an example of the method of using the patch is as follows. The patch according to the present invention is first attached on a polynucleotide array substrate having an immobilized probe polynucleotide, so as to form a reaction chamber. Then, a hybridization reaction mixture containing a target sample is injected into the reaction chamber, and hybridization is undergone under constant conditions. After completing hybridization, the resultant is washed with a washing fluid and sufficiently dried, and then the level of hybridization is determined by scanning via a scanner.

An embodiment of the present invention will now be described in greater detail with reference to the accompanying drawings. FIG. 3 is a side view of the patch for the microarray reaction chamber of the present invention. Referring to FIG. 3, the patch is composed of a support means 10 and an adhesive means 9 attached to the support means 10. The adhesive means 9 is composed of a first adhesive layer 13 and a second adhesive layer 15. In the first adhesive layer 13, a first adhesive material 11 is deposited on upper and lower surfaces of an adhesive means support 12. In the second adhesive layer 15, the first adhesive material 11 is deposited on an upper surface of another adhesive means support 12 and a second adhesive material 14 is deposited on a lower surface thereof. The adhesive means 9 can be attached to the support means 10 by the first adhesive material 11 deposited on the upper surface of the first adhesive layer 13. Also, the adhesive means 9 can be attached to the microarray substrate by the second adhesive material 14 deposited on the lower surface of the second adhesive layer 15. The patch is provided with a reaction chamber-forming portion 22, which forms a part of the reaction chamber when being attached to the microarray substrate. The reaction chamber-forming portion 22 may be formed by cutting out the adhesive means 9 in a regular form. The thickness of the adhesive means 9 may vary depending on the number of adhesive means supports 12 used. Thus, the thickness of the adhesive means 9 can be controlled by adjusting the number of adhesive means supports 12, thereby controlling the capacity of the reaction chamber-forming portion 22.

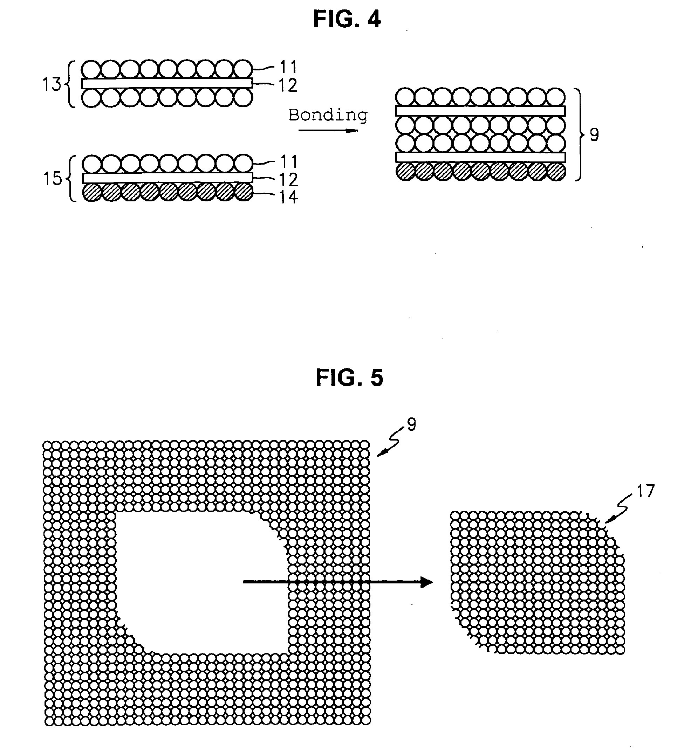

The patch for the microarray reaction chamber according to an embodiment of the present invention may be manufactured, for example, according to the following process. The first adhesive material 11 is first deposited on the upper and the lower surfaces of one adhesive means support 12 so as to form the first adhesive layer 13. The first adhesive material 11 is further deposited on the upper surface of another adhesive means support 12 and the second adhesive material 14 is deposited on the lower surface of the other adhesive means support 12, so as to form the second adhesive layer 15. Then, the first adhesive layer 13 and the second adhesive layer 15 are joined via the adhesive strength between the first adhesive material, so as to form the adhesive means 9 (FIG. 4). Then, a part of the adhesive means 9 is cut out, in proper size and form, to form the reaction chamber-forming portion 22 (FIG. 5). The adhesive means 9 provided with the reaction chamber-forming portion 22 is joined with the support means 10 provided with a sample inlet, thereby forming the patch of the present invention.

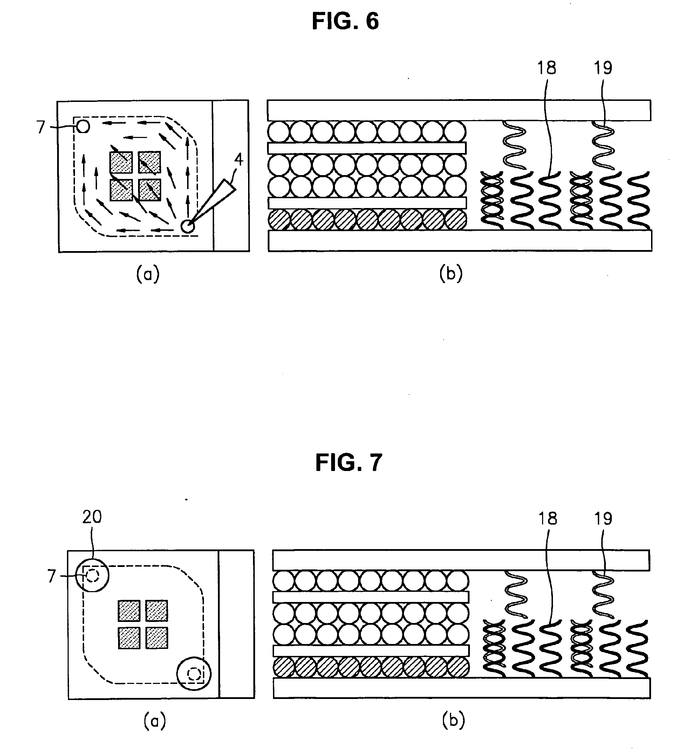

FIGS. 6 and 7 illustrate examples of the method of using the patch of the present invention. FIG. 6A is a plan perspective view illustrating the patch of the present invention attached to a polynucleotide microarry substrate. A reaction chamber is formed by attaching the patch of the present invention to the microarray substrate. When injecting a reaction mixture into the reaction chamber through a sample inlet, the sample is distributed in the reaction chamber as shown by the arrows. FIG. 6B is a partial side view of the patch of FIG. 6A. Then, the patch is closed using a tape 20 of the sample inlet 7 and incubated under hybridization conditions, for example, at 37°C for 16 hours (FIG. 7A). FIG. 7B is a partial side cross-sectional view of the reaction chamber when injecting a sample into the reaction chamber formed by attaching the patch of the present invention to a microarray substrate. The reaction chamber is formed by joining the adhesive means of the patch with the substrate, using the second adhesive material. A probe polynucleotide 18 immobilized on the substrate is hybridised with a target polynucleotide 19 in the sample. During hybridization, the sample must not leak out from the patch. The second adhesive material must be easily detached and must not leave residues on the substrate. To satisfy the above requirements, the second adhesive material may have an adhesive strength of 12-25 oz/in (according to ASTM D3330 Method), based on an adhesive strength to steel. Also, the first adhesive material has to strongly adhere to the support means to be later detached when removing the patch. The first adhesive material may have an adhesive strength of 45 oz/in (according to ASTM D3330 Method), based on an adhesive strength to steel.

The present invention will be described in greater detail with reference to the following examples. The following examples are for illustrative purposes and are not intended to limit the scope of the invention.

### Examples

### Example 1: Effects of the adhesive strengths of a first adhesive material and a second adhesive material on characteristics of a patch

In the present Example, 9495 MP 3M tape™ of 120 *µ*m thickness, which has a first adhesive material deposited on upper and lower surfaces, was used as a first adhesive layer. 9425PC 3M tape^{TM} having a upper surface coated with the first adhesive material having an adhesive strength of 45 oz/in and a lower surface coated with a second adhesive material having an adhesive strength of 12 oz/in was used as a second adhesive layer. These two adhesive layers were first joined together and then joined with a support means made of polycarbonate, which had a thickness of 200 µm and was provided with a sample inlet, thereby forming a patch for a microarray chamber.

Then, the patch was attached to a microarray substrate so as to form a reaction chamber. A sample containing a target polynucleotide was injected into the reaction chamber through the sample inlet and hybridization was undergone at 37°C for 16 hours. Then, the patch was detached. Sample leakage rate during hybridization, detaching satisfaction rate, and support means attaching rate were investigated and recorded. Three measurers repeated the measurements 20 times. The sample leakage rate was determined based on loss of the sample due to leakage, using a scanner (GenePix 4000B^{TM}, Axon Instruments, Inc.). The detaching satisfaction rate was an average value of the satisfaction of each measurer when detaching the patch after hybridization. Satisfaction was rated 0 when detaching was impossible and 100 when the patch was easily detached without deforming. If adhesive materials remained when the patch was detached, a satisfaction value between 0 and 100 was assigned. When the first adhesive material did not detached from the support means after hybridization, a value of 100 was assigned as the support means attaching rate.

The obtained results are shown in Table 1 below.

**Table 1**

| Effects of adhesive strength on characteristics of the patch | | | |
|---|---|---|---|
| Adhesive strength (Oz/in) | Sample leakage rate (%) | Detaching satisfaction rate (%) | Support means attaching rate (%) |
| 4 | 100 | 60 | 0 |
| 5 | 100 | 70 | 0 |
| 12 | 0 | 100 | 0 |
| 23 | 0 | 100 | 10 |
| 36 | 0 | 80 | 70 |
| 40 | 0 | 80 | 80 |
| 45 | 0 | 50 | 100 |
| 53 | 0 | 30 | 100 |
| 54 | 0 | 20 | 100 |
| 67 | 0 | 10 | 100 |
| 100 | 0 | 0 | 100 |
| 128 | 0 | 0 | 100 |
| 145 | 0 | 0 | 100 |
| 225 | 0 | 0 | 100 |

Each of values shown in Table 1 is an average of the results obtained from repeating the experiments 20 times. Referring to Table 1, sample leakage did not occur at an adhesive strength of 12 oz/in or greater. Regarding the detaching satisfaction rate, optimum results were obtained at an adhesive strength between 12-23 oz/in. Satisfactory results of the support means attaching rate were obtained at an adhesive strength of 45 oz/in or greater.

In other words, the adhesive strength of the second adhesive material at which sample leakage did not occur and satisfactory detaching was obtained was between 12-23 oz/in. When the first adhesive material had an adhesive strength of 45 oz/in or greater, the patch was not easily detached from the support means. However, the adhesive strength difference between the second adhesive material and the first adhesive material must be 33 oz/in or greater. When this requirement is satisfied, the adhesive strength difference between the adhesive means and the microarray substrate is sufficiently different from the adhesive strength difference between the adhesive means and the support means, thereby obtaining a satisfactory detaching satisfaction rate.

### Example 2

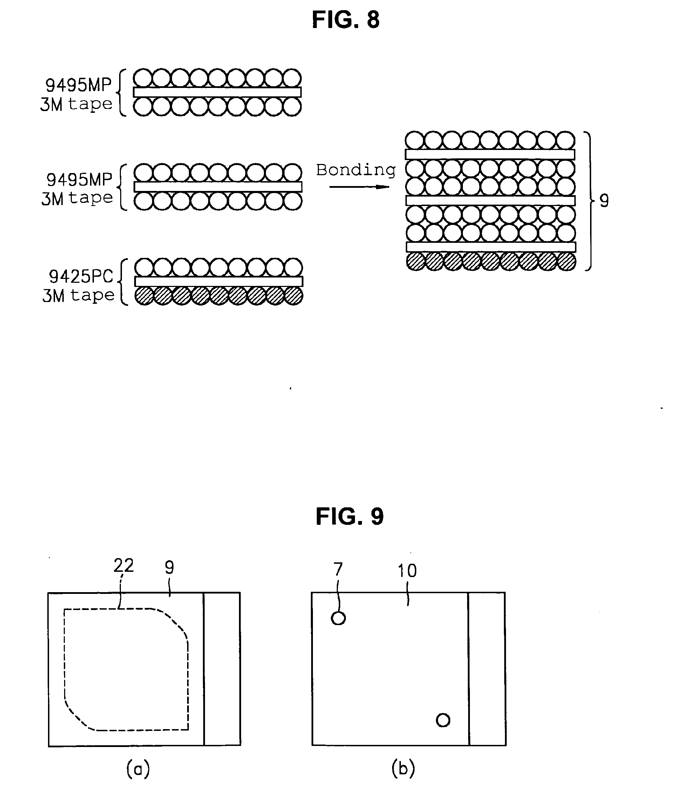

Based on the results of Example 1, 9425PC^{TM} (3M) manufactured by depositing a material with an adhesive strength of 12 oz/in and a material with an adhesive strength of 45 oz/in to both sides of a film was used as a second adhesive layer. 9495MP^{TM} (3M) manufactured by depositing an adhesive material with an adhesive strength of 100 oz/in to both sides of a film, in order to adjust the thickness of the film, was used as a first adhesive layer. A sheet of 9425PC^{TM} and two sheets of 9495MP™ were combined to obtain a joined tape of 400 *µ*m thickness. The joined tape was attached to a polycarbonate film of 200 *µ*m thickness, so as to obtain a patch (FIG. 8). FIG. 9A illustrates the adhesive means in which a space of 14 mm x 14 mm in the obtained adhesive means is cut out to form a chamber-forming portion. FIG. 9B illustrates a polycarbonate support means provided with a sample inlet 7 for injecting a sample. As shown in FIG. 9A, the chamber-forming portion is designed to be streamlined when viewed from above, by eliminating the sharp edges of conventional chambers.

Measurements of sample leakage rate, detaching satisfaction rate, and support means attaching rate were taken on the obtained microarray hybridization reaction chamber. The obtained results are shown in Table 2 below.

**Table 2**

| Characteristics of the patch (average values of 20 measurements) | | | | |
|---|---|---|---|---|
| Characteristics | Reaction temperature | | | |
| | 32°C | 42°C | 52°C | 62°C |
| Sample leakage rate (%) | 0 | 0 | 0 | 0 |
| Detaching satisfaction rate (%) | 100 | 100 | 100 | 100 |
| Support means attaching rate (%) | 100 | 100 | 100 | 100 |

As shown in Table 2, satisfactory results of 0% sample leakage, 100% detaching satisfaction, and 100% support means attaching were obtained.

A test was performed on a polynucleotide array using the microarray reaction chamber. The experimental conditions were as follows.
1) Probe polynucleotide: SEQ ID No. 1
   Target polynucleotide: SEQ ID No. 2, 500 pM
   Spot pitch: 300 *µ*m, spot diameter: 160 *µ*m, number of spots: 528
2) Hybridization time and temperature: 16 hours and 32°C
3) Washing time: I step - 5 minutes with 6X SSC, II step - 5 minutes with 3X SSC
4) PMT 573, measured with Greenmode at a wavelength of 532 nm (Scanner GenePix 4000B, Axon Instruments, Inc.)

Scanning yielded an average intensity of fluorescence of 9655.31, indicating that the patch of the present invention can perform a function as a reaction chamber of a polynucleotide array.

A patch for a reaction chamber of a microarray according to the present invention can prevent a sample from leaking and be easily detached from a microarray substrate without separating an adhesive means and a support means. Also, the capacity of the reaction chamber can be easily adjusted.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A patch for a reaction chamber of a microarray, which includes a support means provided with a sample inlet and an adhesive means adhering to the support means and providing an adhesive strength capable of being attached to a microarray substrate and has a chamber-forming portion defined by the support means and the adhesive means,
wherein the adhesive means has stacked adhesive means supports having both surfaces to which an adhesive material is deposited and a first adhesive material located between the adhesive means support and the adhesive means support or between the adhesive means support and the support means has 33 oz/in greater adhesive strength (according to ASTM D3330 Method), based on an adhesive strength to steel, than a second adhesive material located between the patch and the microarray substrate.

2. The patch of claim 1, wherein the support means is composed of a hydrophobic material having a contact angle of 80° or greater.

3. The patch of claim 2, wherein the hydrophobic material is a hydrophobic plastic or a hydrophobically-modified silicone.

4. The patch of claim 2, wherein the hydrophobic material is selected from the group consisting of polyethylene, polypropylene, polycarbonate, polystyrene, polymethylmethacrylate (PMMA), polydimethylsiloxane (PDMS), polychlorinated biphenyl (PCB), hydrophobically-modified silicon wafer, hydrophobically-modified ITO (indium tin oxide) glass, and a combination thereof.

5. The patch of claim 1, wherein the adhesive means support is selected from the group consisting of polyethyleneterephthalate (PET), polyethylene, and polyester.

6. The patch of claim 1, wherein the first adhesive material has an adhesive strength of 45 oz/in or greater.

7. The patch of claim 1, wherein the second adhesive material has an adhesive strength of 12-25 oz/in.

8. The patch of claim 1, wherein the chamber-forming portion has a steamline form.

9. A method of using the patch of any one of claims 1 through 8, the method comprising:
attaching the patch of any one of claims 1 through 8 on a microarray substrate having an immobilized biomolecule, so as to form a reaction chamber; and
injecting a reaction mixture into the reaction chamber so as to perform a reaction.

10. The method of claim 9, wherein the microarray is a protein or polynucleotide array.


SEQUENCE LISTING

<110>    Samsung Electronis Co. Ltd.

<120>    A patch for microarray reaction chamber having adhesive means
         support and two or more adhesion materials

<130>    PN052197

<160>    2

<170>    KopatentIn 1.71

<210>    1
<211>    15
<212>    DNA
<213>    Artificial Sequence

<220>
<223>    probe polynucleotide

<400>    1

tgttctcttg tcttg                    15

<210>    2
<211>    15
<212>    DNA
<213>    Artificial Sequence

<220>
<223>    target polynucleotide

<400>    2

acaagagaac agaac                    15